# EUROPEAN PATENT SPECIFICATION

(11) **EP 3 119 252 B1**
(45) Date of publication and mention of the grant of the patent: **05.12.2018**
(21) Application number: 15765609.1
(22) Date of filing: 18.03.2015
(51) Int. Cl.: A47K 11/04, A47K 11/06, A61B 10/00

(54) **BEDPAN SYSTEM**
BETTPFANNENSYSTEM
SYSTÈME DE BASSIN HYGIÉNIQUE

(30) Priority: 19.03.2014 DK 201470134
(43) Date of publication of application: 25.01.2017
(73) Proprietor: Goloo ApS, 7000 Fredericia (DK)
(72) Inventor: RON, Izac, DK-7000 Fredericia (DK)
(74) Representative: Olesen, Birthe Bjerregaard
(86) International application number: PCT/DK2015/050054
(87) International publication number: WO 2015/139707

(56) References cited:
- US-A- 3 588 921
- US-A- 4 759 086
- US-A- 5 144 698
- US-A1- 2007 245 486
- US-A1- 2009 119 828
- US-A1- 2009 119 828
- US-A1- 2010 175 178
- US-A1- 2010 175 178
- US-B1- 7 975 326

## Description

### Field of the Invention

The present invention concerns a bedpan system and a method for using a bedpan system suited for use in a bedpan chair, wherein the bedpan chair includes a seat with an outer periphery, the seat having an opening substantially and functionally corresponding to the opening in a toilet seat on a traditional water-flushed toilet, and wherein the bedpan system is constituted by a bedpan bag with a top, a bottom, an outer side and an inner side, the bedpan bag including at least one receptacle portion between the inner side and the bottom for receiving faeces and/or urine, wherein the bedpan bag further, and that the bedpan bag further includes a tear-off edge for separating the receptacle portion from the fixing part, the tear-off edge being closer to the fixing part than to the bottom of the receptacle portion, and that the tear-off edge is prepared as perforation or other pre-pressed deformation of the bag.

The present invention also concerns a single use bedpan bag for use in the above mentioned system and/or method.

### Background of the Invention

In connection with caring for persons that are sick or for other reasons unable to use a toilet, it is commonly known to use a bedpan chair in which bedpans of steel or hard plastic can be mounted, and which are to be handled, emptied and cleaned after use. Handling as well as emptying and cleaning of these bedpans is very uninteresting/unwanted, unhygienic, time-consuming and not the least space-demanding as appreciable amounts of space are required in the sluice rooms arranged for the purpose in hospitals and other places caring for persons that cannot manage a toilet visit by themselves. The types of bedpans in use are standardised for the greater part or made such that they can be handled in the same cleaning and sterilising equipment.

Under certain circumstances, such bedpans are used for bedridden persons, whereas in other cases where the person can get out of bed but cannot manage the visit to the toilet, so-called bedpan chairs are used where a bedpan is placed under a seat in the bedpan chair which then can be used as a toilet. These bedpan chairs are in principle common chairs, though often with wheels under the legs, and where an opening is formed in the seat largely corresponding to the opening in a usual toilet seat. The bedpan, which just can be a standardised bedpan, is then provided under this seat prior to use. Provision of the bedpan typically occurs from the back side of the bedpan chair in that the bedpan is disposed in rails arranged for the purpose under the seat.

Irrespective of the bedpan being used in one or the other way, it associated with the same inexpediency. For the user as well for the care assistants, this is a transgressive operation which has been left largely unchanged for many years. The development that has occurred in connection with bedpan chairs has been on the chair itself, which in a modern variant is provided with various adjustment options. Moreover, special washing machines have been developed for the various bedpan types. Finally, particularly the space-consuming functions like cleaning, sterilising and storage of space-demanding bedpans have resulted in bedpan systems as mentioned in the introduction and as known from US 7975326.

US 3588921 A discloses a toilet mounted disposable stool specimen collector. The stool collector comprises a toilet cover and a stool collector bag that is arranged so as to collect the stool only. A front end opening between the toilet seat cover part and the stool collector bag allows any urine to pass directly into the toilet.

US 2007/245486 A discloses another toilet mounted collector bag for use together toilet seat cover that is divided in the front. The collector bag, as well as comprising all of the features of the preamble of claim 1, comprises a toilet seat cover part that is arranged on the toilet seat by sliding the two half parts of the seat into channels made in the toilet seat cover part of the collector bag.

However, there is still an expressed desire for cleaning/drying the user/patient that is rendered difficult by the prior art embodiments of bedpan systems.

With regard to bedpans for bedridden persons, so-called disposable bedpans have appeared on the market where an air ring in the bedpan is inflated before use. These disposable bedpans are closed and disposed of after use. The company Goloo makes and markets such bedpans. There is thus a partial solution to the problem with the traditional bedpans, but there is still a need for space and equipment for handling the old well-known solution.

### Object of the Invention

It is the object of the invention to indicate a bedpan system for use in bedpan chairs where the bedpan is easy to place and use, and where the bedpan together with faeces and/or urine can be removed in part, the user/patient can be cleaned/dried, and toilet paper/wet wipes and the remaining part of the bedpan bag can be removed after use, whereby an entirely low status, time-consuming and space-demanding task can be performed in a markedly easier way and more hygienically than by the prior art solutions.

### Description of the Invention

As mentioned in the introduction, the invention concerns a bedpan system. The bedpan system according to the invention is constituted by a bedpan chair and a bed pan bag with a top, a bottom, an outer side and an inner side, the bedpan bag including at least one receptacle portion between the inner side and the bottom for receiving faeces and/or urine and the bedpan bag further includes a tear-off edge for separating the receptacle portion from the fixing part, the tear-off edge being closer to the fixing part than the bottom of the receptacle portion, and where the tear-off edge, for example, is prepared as perforation or other pre-pressed deformation of the bag. The system is special in that the bedpan bag further, preferably at the top, includes a fixing part that includes fixing means in the form of adhesives for fixing the fixing part to the bedpan chair seat. The system is further special in that the bedpan bag, when placed in the opening of the seat of the bed pan chair, covers the entire opening in the seatand

The invention also concerns a single use bedpan bag, wherein the bedpan bag is provided with a top, a bottom, an outer side and an inner side, the bedpan bag including at least one receptacle portion between the inner side and the bottom for receiving faeces and/or urine and that the bedpan bag further includes a tear-off edge for separating the receptacle portion from the fixing part, the tear-off edge being closer to the fixing part than to the bottom of the receptacle portion, and that the tear-off edge is prepared as perforation or other pre-pressed deformation of the bag. The bedpan bag is special in that he bedpan bag further, preferably at the top, includes a fixing part including fixing means in the form of adhesives for fixing the fixing part to the bedpan chair seat. The bedpan bag is further special in that the bedpan bag, when placed in the opening of the seat of the bed pan chair, covers the entire opening in the seat.

By such a bedpan bag and bedpan system there is achieved the significant and obvious advantage that the old-fashioned/traditional bedpans that were reused after cleaning and sterilisation can entirely be dispensed with. By a bedpan system according to the invention there is thus no longer any use for a sluice room arranged for handling bedpans. A bedpan bag according to the invention is a disposable article, and in a preferred variant made of a material which readily can be stored in a box or a cassette until the bedpan bag is to be used. It is so that the old space-demanding bedpans can be completely dispensed with and at the same time substituted by bedpan bags according to the invention which do not take up more than a fraction of the previous solution. A dispenser with bedpan bags can advantageously be provided close to a user such that called care assistants do not need to fetch a bedpan from a storage site as was the case previously. Moreover, as mentioned there is no subsequent work with cleaning etc. Basically, this is a practical, economical and not the least hygienic solution compared with hitherto used, prior art solutions.

A bedpan bag can readily be arranged in the opening of the seat on a bedpan chair of the known types, and the fixing means, typically in the form of one or more adhesive strips or spots with adhesive disposed along the top of the bedpan bag at the fixing part, are activated. Activation typically occurs by removing one or more protective films from the adhesive strip itself. The adhesive strip will typically be arranged spaced apart from the upper top on the bedpan bag and along its fixing part. The adhesive strip is pressed against the seat on the bedpan chair, and the bedpan bag is ready for use. Protective film from the fixing means can advantageously be disposed of in the bedpan bag itself before using it as bedpan.

When the user/patient has relieved themselves, the bedpan bag can be divided as the receptacle portion is torn off the fixing part where the tear-off edge is located, after which the user/patient can be cleaned/dried much more easily by the care assistants. When the user/patient is removed from the bedpan chair, the fixing part of the bedpan bag can be released from the bedpan chair, and the bedpan bag including toilet paper and fixing part can finally be closed with closing means adapted for the purpose, after which the bedpan bag with contents is disposed of as waste.

In a variant of a bedpan system according to the invention, a bedpan bag can advantageously be designed such that the fixing part further includes a cover part with a size and shape substantially corresponding to the size and shape of the seat. In this way the entire seat of the bedpan chair is covered, and a hygienic solution is achieved. In such a variant of a bedpan bag the fixing part has a somewhat larger extent and therefore not only covers a part of the edge of the bedpan chair along the seat, but on the whole the entire seat. A user will therefore not only sit on a fixing part along the edge of the opening in the seat but on the mentioned covering part itself, which in some cases can be advantageous.

A bedpan system according to the invention can be adapted such that the fixing part with fixing means is adapted for fixing the fixing part substantially along the opening in the seat.

Another variant of a bedpan system according to the invention can, however, be adapted with the fixing part with fixing means which are arranged for fixing the fixing part substantially along the outer periphery of the seat. Such a variant is of course suited for a bedpan bag where the seat is covered by a cover part as mentioned above.

In a preferred variant of a bedpan system according to the invention, the bedpan bag can include closing means in the form of at least one tape or band, where the at least one tape or band is fastened to the bedpan bag preferably at the outer side of the bedpan bag and near the top of the bedpan bag. By one or two tapes or bands it is possible to tie up the opening of the bedpan bag after use. This is readily done by gathering the top of the bedpan bag and by using the mentioned bands or tapes for tying around the gathered top.

In a variant of a bedpan system according to the invention, the bedpan bag can include closing means in the form of at least one tape or band, where the at least one tape or band is arranged in a drawstring hem, the drawstring hem arranged at or near the top of the bag, where the at least one tape or band is accessible from the outer side of the bedpan bag. By such a solution is obviously achieved the same advantages as when a drawstring hem with a tape is used, namely a rapid and simple solution by which a person simply pulls the tape while simultaneously keeping the bedpan bag fixed, whereby the bedpan bag opening is drawn together and closed. The used band or tape can subsequently e.g. be tied around the top of the bag in order to secure the closure.

The bedpan bag can, however, also be closed with one or more adhesive strips, e.g. the same adhesive strips used for fixing the bedpan bag to the seat in the bedpan chair. Alternatively, a bedpan bag for a bedpan system according to the invention can be made with a so-called "reclosing" arrangement as known from plastic bags and other types of packing where two parts of a closing device are brought into mutual engagement.

In a variant of a bedpan system according to the invention, the bedpan bag can be made of at least two layers of a plane web material, e.g. a plastic or paper material, where the web material is joined along at least one edge, thus forming the receptacle portion. The bedpan bag can be joined at one side, two sides and at the bottom but can be folded as well such that the fold constitutes an edge. In a preferred variant, the bedpan bag is provided with two sides, a bottom and a top, where the bottom is constituted by an edge, but a bedpan bag can also be made with a more or less pointed or round bottom part. The bedpan bag will typically be made of a suitable plastic material, e.g. polyethylene, but it can also be made of other types of plastic or of a paper material with suitable tightness, or coated with wax or other liquid-repelling material. A bedpan bag according to the invention can furthermore be made of a multi-layered material where one or more layers are of plastic, paper or other suitable material.

A particularly preferred variant of a bedpan system according to the invention can be designed such that the fixing edge of the bedpan bag includes a number of slits, preferably two, three or four slits, the slits extending from the top of the bedpan bag and towards the receptacle portion. These slits facilitate mounting of the bedpan bag in the opening of the seat on the bedpan chair as the fixing part is more easily folded out to the side. Hereby is achieved a more smooth positioning of the fixing part on the seat, and thereby a more comfortable use of the bedpan chair.

A bedpan system according to the invention can advantageously be with a bedpan bag that includes an insert of super-absorbing polymer (SAP). Faeces and not the least urine are hereby fixed in the bedpan bag, and easier handling is achieved. The said insert of SAP can be fastened in the bedpan bag, ensuring that the insert always has the right and intended position, but it may also be a loose insert that can be taken out before using the bedpan bag. This can be particularly advantageous if one or more urine samples are to be taken out for further analysis.

As mentioned in the introduction, the invention also concerns a method for using a bedpan system suited for use in a bedpan chair, the bedpan chair including a seat with an opening, the opening substantially and functionally corresponding to the opening in a toilet seat on a traditional water-flushed toilet.

Such a method includes at least the following steps:
- placing a bedpan bag in the opening of the seat of a bedpan chair, the bedpan bag at least including a top, a bottom, an outer side and an inner side, wherein a receptacle portion is provided between the inner side and the bottom, and wherein the bedpan bag further and preferably at the top includes a fixing part;
- fixing the fixing part on the seat of the bedpan chair by fixing means adapted for the purpose, preferably self-adhesive fixing means, whereby the bedpan bag covers the entire opening in the seat when fixed to the bedpan seat;
- a user using the bedpan chair;
- the bedpan bag being divided as the receptacle portion is torn off the fixing part where the tear-off edge is disposed;
- a user being cleaned/dried;
- a user going away/being removed from the bedpan chair;
- the fixing part of the bedpan bag being released from the bedpan chair;
- the bedpan bag being closed with closing means adapted for the purpose;
- the bedpan bag with contents being disposed of as waste.

Before the bedpan bag is closed, toilet paper and fixing part can be put therein as well such that most of or all of the waste can be collected in the bedpan bag, after which the bedpan bag is closed and disposed of. Toilet paper and fixing part may of course be disposed of separately as well and in other ways and therefore do not need to go into the bedpan bag before closing and disposal thereof.

By this method is achieved a marked relief in work processes compared with the hitherto used systems where handling, cleaning and hygiene were causing some challenges. By a method as indicated here there is achieved a very simplified solution where a disposable solution is applied, where the solution is hygienic and where the solution is time-saving and far less transgressive for a user as well as for care assistants due to the above mentioned distinctions.

### Description of the Drawing

The invention is described in the following with reference to the drawing, wherein:
- Fig. 1: shows a typical bedpan chair;
- Fig. 2: shows a bedpan chair with a bedpan bag;
- Fig. 3: shows a bedpan bag as seen from the side;
- Fig. 4: shows a bedpan bag isometrically and slightly open.

In the explanation of the Figures, identical or corresponding elements will be provided with the same designations in different Figures. Therefore, an explanation of all details will not be given in connection with each single Figure/embodiment as well as all there will not necessarily be provided an explanation of all shown designations.

### List of designations:

- 1: bedpan chair
- 2: seat
- 3: opening in seat
- 4: bedpan bag
- 5: top of bedpan bag
- 6: fixing part
- 7: slits in fixing part
- 8: bottom of bedpan bag
- 9: receptacle portion
- 10: closing means
- 11: fixing means
- 12: outer side of bedpan bag
- 13: bottom edge of bedpan bag
- 14: side edge of bedpan bag
- 15: inner side of bedpan bag
- 16: flaps at fixing part
- 17: tear-off edge

### Detailed Description of Embodiments of the Invention

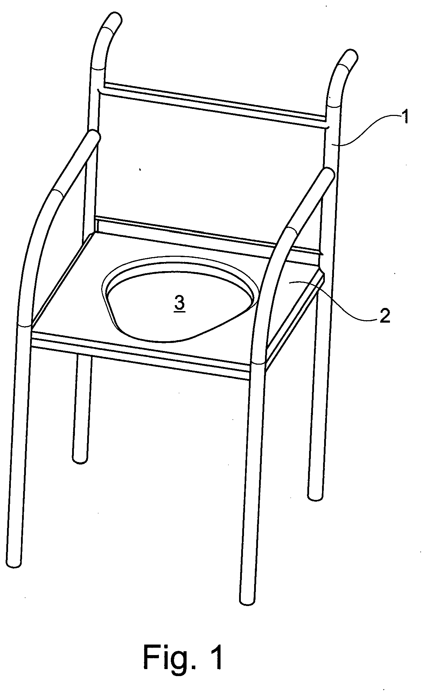

In Fig. 1 appears a typical bedpan chair 1 with a seat 2 in which an opening 3 is arranged that substantially and functionally corresponds to the opening in a traditional toilet seat.

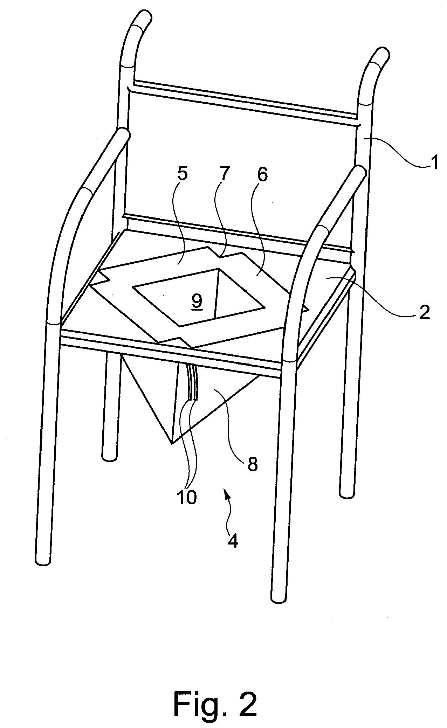

Fig. 2 shows the same bedpan chair 1 as Fig. 1, but here with a bedpan bag 4 mounted in the opening 3. At the top 5 of the bedpan bag is provided a fixing part 6 shown here folded and flush with the seat 2. At the fixing part 6 are seen slits 7 by which the folding of the fixing part 6 is facilitated and a more smooth mounting can take place. The receptacle portion 9 of the bedpan bag is shown at the centre of the opening 3. The bottom 8 of the bedpan bag appears under the seat 2, and immediately under the seat 3 are seen the closing means 10 in the form of two bands or tapes which can be used for closing the bedpan bag 4 after use.

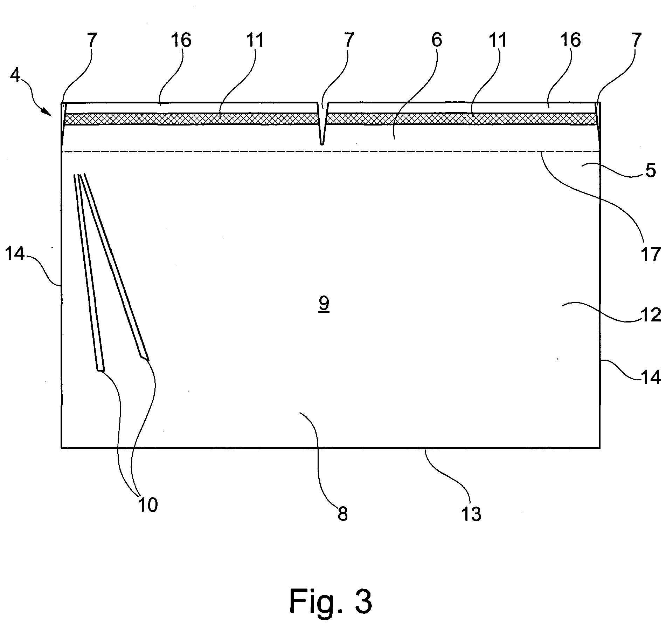

I Fig. 3 is seen the outer side 12 of a bedpan bag 2 where bottom edge 13 as well as side edges 14 are shown. The fixing means 11 are seen at the top 5 of the bedpan bag, here in the form of an adhesive strip extending along the fixing part 6, but interrupted by the slits 7 in the fixing part 6 forming the flaps 16 at the fixing part 6. A tear-off edge 17 closer to the fixing part 6 than to the bottom 8 of the receptacle portion is marked as a broken line, thus indicating where it is possible to separate the receptacle portion 9 from the fixing part 6.

At the outer side 12 of the bedpan bag are also seen the above described closing means 10 in the form of two tapes or bands that e.g. are made of the same or a different plastic material as the bedpan bag 4.

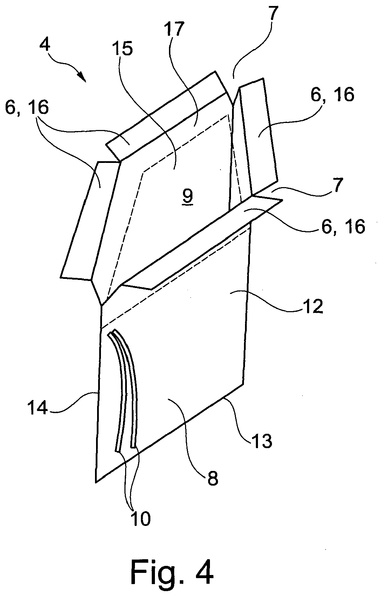

Finally, in Fig. 4 is seen a bedpan bag 4 in isometric view and slightly open. In this Figure appears the receptacle portion 9 which is delimited by the inner side 15, the bottom edge 13 and the side edges 14. At the top 5 of the bedpan bag, the receptacle portion is encircled by the fixing part 6 which is here more or less divided into flaps 16 due to the slits 7, and where a tear-off edge 17 closer to the fixing part 6 than to the bottom 8 of the receptacle portion is also marked as a broken line, indicating where it is possible to separate the receptacle portion 9 from the fixing part 6.

## Claims

1. A single use bedpan bag (4) for use in a bed pan system comprising said bag (4) and a bedpan chair (1), wherein the bedpan chair (1) includes a seat (2) with an outer periphery, the seat having an opening (3) substantially and functionally corresponding to the opening in a toilet seat on a traditional water-flushed toilet, wherein the bedpan bag is provided with a top, (5) a bottom (8), an outer side (12) and an inner side (15), the bedpan bag (4) including at least one receptacle (9) portion between the inner side (15) and the bottom (8) for receiving faeces and/or urine, wherein the bedpan bag (4) further includes, preferably at the top (5), a fixing part (6), and further includes a tear-off edge (17) for separating the receptacle portion (9) from the fixing part (6), the tear-off edge (17) being closer to the fixing part (6) than to the bottom (8) of the receptacle portion (9), and that the tear-off edge (17) is prepared as perforation or other pre-pressed deformation of the bag, the bedpan bag (4) being configured such that when placed in the opening (3) of the seat (2) of the bed pan chair (1), it will cover the entire opening in the seat, **characterised in that** the fixing part (6) includes fixing means (11) in the form of adhesives for fixing the fixing part (6) to the bedpan chair seat (2).

2. A bedpan system comprising the single use bedpan bag according to claim 1 and a bedpan chair (1), wherein the bedpan chair (1) includes a seat (2) with an outer periphery, the seat having an opening (3) substantially and functionally corresponding to the opening in a toilet seat on a traditional water-flushed toilet, and wherein the bedpan bag (4) when placed in the opening (3) of the seat (2) of the bed pan chair (1) covers the entire opening (3) in the seat.

3. A bedpan bag according to claim 1, **characterised in that** the fixing part (6) further includes a cover part with a size and shape substantially corresponding to the size and shape of the seat (2).

4. A bedpan bag according to either of claims 1 or 3, **characterised in that** the fixing part (6) with fixing means (11) is adapted for fixing the fixing part (6) substantially along the opening (3) in the seat (2).

5. A bedpan bag according to any of claims 1, 3 or 4, **characterised in that** the fixing part (6) with fixing means (11) is adapted for fixing the fixing part (6) substantially along the outer periphery of the seat (2).

6. A bedpan bag according to any of claims 1 or 3 to 5, **characterised in that** the bedpan bag (4) includes closing means (10) in the form of at least one tape or band, where the at least one tape or band is fastened to the bedpan bag (4) preferably at the outer side (12) of the bedpan bag and near the top (5) of the bedpan bag.

7. A bedpan bag according to any of claims 1 or 3 to 6, **characterised in that** the bedpan bag (4) includes closing means (10) in the form of at least one tape or band, where the at least one tape or band is arranged in a drawstring hem, the drawstring hem arranged at or near the top (5) of the bag, where the at least one tape or band is accessible from the outer side (12) of the bedpan bag (4).

8. A bedpan bag according to any of claims 1 or 3 to 7, **characterised in that** the bedpan bag is made of at least two layers of a plane web material, e.g. a plastic or paper material, where the web material is joined along at least one edge, thus forming the receptacle portion.

9. A bedpan bag according to any of claims 1 or 3 to 8, **characterised in that** a fixing edge of the bedpan bag includes a number of slits (7), preferably two, three or four slits, the slits (7) extending from the top of the bedpan bag (4) and towards the receptacle portion (9).

10. A bedpan bag according to any of claims 1 or 3 to 9, **characterised in that** the bedpan bag (4) includes an insert of super-absorbing polymer (SAP).

11. A method for using a bedpan bag according to any of claims 1 or 3 to 10 in a bed pan system according to claim 2, **characterised in that** the method includes at least the following steps:
- placing the bedpan bag (4) in the opening (3) of the seat (2) of the bedpan chair (1);
- fixing the fixing part (6) on the seat (2) of the bedpan chair by fixing means (11) adapted for the purpose, preferably self-adhesive fixing means (11), whereby the bedpan bag (4) covers the entire opening (3) in the seat (2) when fixed to the bedpan seat (2);
- a user using the bedpan chair;
- the bedpan bag (4) being divided as the receptacle portion (9) is torn off the fixing part (6) where a tear-off edge(17) is disposed;
- a user being cleaned/dried;
- a user going away/being removed from the bedpan chair (1);
- the fixing part (6) of the bedpan bag (4) being released from the bedpan chair (1);
- the bedpan bag (4) being closed with closing means (10) adapted for the purpose;
- the bedpan bag (4) with contents being disposed of as waste.

## Patentansprüche

1. Einweg-Bettpfannenbeutel (4) zur Verwendung in einem Bettpfannensystem, das den Beutel (4) und einen Bettpfannenstuhl (1) umfasst, wobei der Bettpfannenstuhl (1) einen Sitz (2) mit einem äußeren Umfang umfasst, wobei der Sitz eine Öffnung (3) aufweist, die im Wesentlichen und funktionell der Öffnung in einem Toilettensitz auf einer traditionellen wassergespülten Toilette entspricht, wobei der Bettpfannenbeutel mit einer Oberseite (5), einem Boden (8), einer äußeren Seite (12) und einer inneren Seite (15) bereitgestellt wird, wobei der Bettpfannenbeutel (4)zumindest einen Aufnahmeabschnitt (9) zwischen der inneren Seite (15) und dem Boden (8) zur Aufnahme von Fäkalien und/oder Urin beinhaltet, wobei der Bettpfannenbeutel (4) ferner, vorzugsweise an der Oberseite (5) ein Befestigungsteil (6) beinhaltet und ferner eine Abreißkante (17) zum Trennen des Aufnahmeabschnitts (9) von dem Befestigungsteil (6) beinhaltet, wobei die Abreißkante (17) näher an dem Befestigungsteil (6) als an dem Boden (8) des Aufnahmeabschnitts (9) liegt, und dass die Abreißkante (17) als Perforation oder andere vorgepresste Verformung des Beutels ausgebildet ist, wobei der Bettpfannenbeutel (4) so konfiguriert ist, dass er, wenn er in die Öffnung (3) des Sitzes (2) des Bettpfannenstuhls (1) eingesetzt wird, die gesamte Öffnung des Sitzes abdeckt, **dadurch gekennzeichnet, dass** das Befestigungsteil (6) Befestigungsmittel (11)in Form von Klebstoffen zur Befestigung des Befestigungsteils (6) am Sitz (2) des Bettpfannenstuhls beinhaltet.

2. Bettpfannensystem, umfassend den Einweg-Bettpfannenbeutel nach Anspruch 1 und einen Bettpfannenstuhl (1), wobei der Bettpfannenstuhl (1) einen Sitz (2) mit einem äußeren Umfang beinhaltet, wobei der Sitz eine Öffnung (3) aufweist, die im Wesentlichen und funktionell der Öffnung in einem Toilettensitz auf einer traditionellen wassergespülten Toilette entspricht, und wobei der Bettpfannenbeutel (4), wenn er in die Öffnung (3) des Sitzes (2) des Bettpfannenstuhls (1) eingesetzt wird, die gesamte Öffnung (3) des Sitzes bedeckt.

3. Bettpfannenbeutel nach Anspruch 1, **dadurch gekennzeichnet, dass** das Befestigungsteil (6) ferner ein Abdeckteil mit einer Größe und Form beinhaltet, die im Wesentlichen der Größe und Form des Sitzes (2) entspricht.

4. Bettpfannenbeutel nach einem der Ansprüche 1 oder 3, **dadurch gekennzeichnet, dass** das Befestigungsteil (6) mit Befestigungsmitteln (11) zum Befestigen des Befestigungsteils (6) im Wesentlichen entlang der Öffnung (3) im Sitz (2) angepasst ist.

5. Bettpfannenbeutel nach einem der Ansprüche 1, 3 oder 4, **dadurch gekennzeichnet, dass** das Befestigungsteil (6) mit Befestigungsmitteln (11) zum Befestigen des Befestigungsteils (6) im Wesentlichen entlang des äußeren Umfangs des Sitzes (2) angepasst ist.

6. Bettpfannenbeutel nach einem der Ansprüche 1 oder 3 bis 5, **dadurch gekennzeichnet, dass** der Bettpfannenbeutel (4) Verschlussmittel (10) in Form von zumindest einem Klebeband oder Band beinhaltet, wobei das zumindest eine Klebeband oder Band an den Bettpfannenbeutel (4), vorzugsweise an der äußeren Seite (12) des Bettpfannenbeutels, und nahe der Oberseite (5) des Bettpfannenbeutels befestigt ist.

7. Bettpfannenbeutel nach einem der Ansprüche 1 oder 3 bis 6, **dadurch gekennzeichnet, dass** der Bettpfannenbeutel (4) Verschlussmittel (10) in Form von zumindest einem Klebeband oder Band beinhaltet, wobei das zumindest eine Klebeband oder Band in einem Kordelzug angeordnet ist, wobei der Kordelzug am oder nahe der Oberseite (5) des Beutels angeordnet ist, wobei das zumindest eine Klebeband oder Band von der äußeren Seite (12) des Bettpfannenbeutels (4) zugänglich ist.

8. Bettpfannenbeutel nach einem der Ansprüche 1 oder 3 bis 7, **dadurch gekennzeichnet, dass** der Bettpfannenbeutel aus zumindest zwei Schichten eines ebenen Bahnmaterials, z. B. eines Kunststoff- oder Papiermaterials, besteht, wobei das Bahnmaterial entlang zumindest einer Kante verbunden ist und so den Aufnahmeabschnitt bildet.

9. Bettpfannenbeutel nach einem der Ansprüche 1 oder 3 bis 8, **dadurch gekennzeichnet, dass** eine Fixierkante des Bettpfannenbeutels eine Anzahl von Schlitzen (7), vorzugsweise zwei, drei oder vier Schlitze, beinhaltet, wobei sich die Schlitze (7) von der Oberseite des Bettpfannenbeutels (4) und in Richtung des Aufnahmeabschnitts (9) erstrecken.

10. Bettpfannenbeutel nach einem der Ansprüche 1 oder 3 bis 9, **dadurch gekennzeichnet, dass** der Bettpfannenbeutel (4) einen Einsatz aus superabsorbierendem Polymer (SAP) beinhaltet.

11. Verfahren zur Verwendung eines Bettpfannenbeutels nach einem der Ansprüche 1 oder 3 bis 10 in einem Bettpfannensystem nach Anspruch 2, **dadurch gekennzeichnet, dass** das Verfahren zumindest die folgenden Schritte umfasst:
- Platzieren des Bettpfannenbeutels (4) in der Öffnung (3) des Sitzes (2) des Bettpfannenstuhls (1);
- Befestigen des Befestigungsteils (6) am Sitz (2) des Bettpfannenstuhls mit dafür angepassten Befestigungsmitteln (11), vorzugsweise selbstklebenden Befestigungsmitteln (11), wobei der Bettpfannenbeutel (4) die gesamte Öffnung (3) im Sitz (2) abdeckt, wenn er am Bettpfannenstuhl (2) befestigt ist;
- einen Benutzer, der den Bettpfannenstuhl benutzt;
- wobei der Bettpfannenbeutel (4) geteilt wird, während der Aufnahmeabschnitt (9) vom Befestigungsteil (6) abgetrennt wird, wo eine Abreißkante (17) angeordnet ist;
- einen Benutzer, der gereinigt/getrocknet wird;
- einen Benutzer, der vom Bettpfannenstuhl (1) weggeht/entfernt wird;
- wobei das Befestigungsteil (6) des Bettpfannenbeutels (4) von dem Bettpfannenstuhl (1) gelöst wird;
- wobei der Bettpfannenbeutel (4) mit einem für den Zweck angepassten Verschlussmittel (10) verschlossen wird;
- wobei der Bettpfannenbeutel (4) mit dem Inhalt als Abfall entsorgt wird.

## Revendications

1. Sac de bassin hygiénique à usage unique (4) destiné à être utilisé dans un système de bassin hygiénique comprenant ledit sac (4) et une chaise de bassin hygiénique (1), dans lequel la chaise de bassin hygiénique (1) inclut un siège (2) avec une périphérie extérieure, le siège ayant une ouverture (3) correspondant sensiblement et fonctionnellement à l'ouverture d'un siège de toilettes sur des toilettes à chasse d'eau traditionnelles, le sac de bassin hygiénique étant doté d'une partie supérieure (5), d'une partie inférieure (8), d'un côté extérieur (12) et d'un côté intérieur (15), le sac de bassin hygiénique (4) incluant au moins une partie de réceptacle (9) entre le côté intérieur (15) et la partie inférieure (8) pour recevoir les fèces et/ou l'urine, le sac de bassin hygiénique (4) incluant en outre, de préférence au niveau de la partie supérieure (5), une partie de fixation (6), et inclut en outre un bord de coupe (17) pour séparer la partie de réceptacle (9) de la partie de fixation (6), le bord de coupe (17) étant plus proche de la partie de fixation (6) que de la partie inférieure (8) de la partie de réceptacle (9), et que le bord de coupe (17) est préparé en tant que perforation ou une autre déformation pré-pressée du sac, le sac de bassin hygiénique (4) étant configuré de sorte que, lorsqu'il est placé dans l'ouverture (3) du siège (2) de la chaise de bassin hygiénique (1), il recouvrira l'ouverture entière du siège, **caractérisé en ce que** la partie de fixation (6) inclut un moyen de fixation (11) sous forme d'adhésifs pour fixer la partie de fixation (6) au siège de chaise de bassin hygiénique (2).

2. Système de bassin hygiénique comprenant le sac de bassin hygiénique à usage unique selon la revendication 1 et une chaise de bassin hygiénique (1), dans lequel la chaise de bassin hygiénique (1) inclut un siège (2) avec une périphérie extérieure, le siège ayant une ouverture (3) correspondant sensiblement et fonctionnellement à l'ouverture d'un siège de toilettes sur des toilettes à chasse d'eau traditionnelles, et dans lequel le sac de bassin hygiénique (4), lorsqu'il est placé dans l'ouverture (3) du siège (2) de la chaise de bassin hygiénique (1), recouvre l'ouverture entière (3) du siège.

3. Sac de bassin hygiénique selon la revendication 1, **caractérisé en ce que** la partie de fixation (6) inclut en outre une partie de couvercle avec une taille et une forme correspondant sensiblement à la taille et à la forme du siège (2) .

4. Sac de bassin hygiénique selon la revendication 1 ou 3, **caractérisé en ce que** la partie de fixation (6) avec le moyen de fixation (11) est conçue pour fixer la partie de fixation (6) sensiblement le long de l'ouverture (3) du siège (2).

5. Sac de bassin hygiénique selon l'une quelconque des revendications 1, 3 ou 4, **caractérisé en ce que** la partie de fixation (6) avec le moyen de fixation (11) est conçue pour fixer la partie de fixation (6) sensiblement le long de la périphérie extérieure du siège (2).

6. Sac de bassin hygiénique selon l'une quelconque des revendications 1 ou 3 à 5, **caractérisé en ce que** le sac de bassin hygiénique (4) inclut un moyen de fermeture (10) sous forme d'au moins un ruban ou une bande, dans lequel l'au moins un ruban ou une bande est fixé au sac de bassin hygiénique (4) de préférence au niveau du côté extérieur (12) du sac de bassin hygiénique et près de la partie supérieure (5) du sac de bassin hygiénique.

7. Sac de bassin hygiénique selon l'une quelconque des revendications 1 ou 3 à 6, **caractérisé en ce que** le sac de bassin hygiénique (4) inclut un moyen de fermeture (10) sous forme d'au moins un ruban ou une bande, dans lequel l'au moins un ruban ou une bande est agencé dans un ourlet de cordon, l'ourlet de cordon étant agencé au niveau ou près de la partie supérieure (5) du sac, dans lequel l'au moins un ruban ou une bande est accessible à partir du côté extérieur (12) du sac de bassin hygiénique (4).

8. Sac de bassin hygiénique selon l'une quelconque des revendications 1 ou 3 à 7, **caractérisé en ce que** le sac de bassin hygiénique est constitué d'au moins deux couches d'un matériau en bande plane, par exemple un matériau en plastique ou en papier, dans lequel le matériau en bande est joint le long d'au moins un bord, formant ainsi la partie de réceptacle.

9. Sac de bassin hygiénique selon l'une quelconque des revendications 1 ou 3 à 8, **caractérisé en ce qu'**un bord de fixation du sac de bassin hygiénique inclut un nombre de fentes (7), de préférence deux, trois ou quatre fentes, les fentes (7) s'étendant à partir de la partie supérieure du sac de bassin hygiénique (4) et vers la partie de réceptacle (9).

10. Sac de bassin hygiénique selon l'une quelconque des revendications 1 ou 3 à 9, **caractérisé en ce que** le sac de bassin hygiénique (4) inclut un insert de polymère superabsorbant (SAP).

11. Procédé d'utilisation d'un sac de bassin hygiénique selon l'une quelconque des revendications 1 ou 3 à 10 dans un système de bassin hygiénique selon la revendication 2, **caractérisé en ce que** le procédé inclut au moins les étapes suivantes :
- le placement du sac de bassin hygiénique (4) dans l'ouverture (3) du siège (2) de la chaise de bassin hygiénique (1) ;
- la fixation de la partie de fixation (6) sur le siège (2) de la chaise de bassin hygiénique par le moyen de fixation (11) conçu à cet effet, de préférence un moyen de fixation autoadhésif (11), moyennant quoi le sac de bassin hygiénique (4) recouvre l'ouverture entière (3) du siège (2) lorsqu'il est fixé au siège de bassin hygiénique (2) ;
- un utilisateur utilisant la chaise de bassin hygiénique ;
- le sac de bassin hygiénique (4) étant divisé lorsque la partie de réceptacle (9) est arrachée de la partie de fixation (6) où un bord de coupe (17) est disposé ;
- un utilisateur étant nettoyé/séché ;
- un utilisateur s'éloignant/se retirant de la chaise de bassin hygiénique (1) ;
- la partie de fixation (6) du sac de bassin hygiénique (4) étant libérée de la chaise de bassin hygiénique (1) ;
- le sac de bassin hygiénique (4) étant fermé avec un moyen de fermeture (10) conçu à cet effet ;
- le sac de bassin hygiénique (4) ayant une teneur éliminée sous forme de déchets.
